# EUROPEAN PATENT SPECIFICATION

(11) **EP 2 117 635 B1**
(45) Date of publication and mention of the grant of the patent: **02.12.2015**
(21) Application number: 08743826.3
(22) Date of filing: 13.03.2008
(51) Int. Cl.: A61N 2/02, A61B 17/62

(54) **ENCOMPASSING EXTERNAL FIXATION DEVICE WITH INCORPORATED PEMF COIL**
EXTERNER FIXIERRAHMEN MIT INTEGRIERTER PEMF-SPULE
DISPOSITIF DE FIXATION EXTERNE PÉRIPHÉRIQUE À BOBINE PEMF INCORPORÉE

(30) Priority: 15.03.2007 US 894956 P
(43) Date of publication of application: 18.11.2009
(73) Proprietor: Amei Technologies. Inc., Wilmington DE 19899 (US)
(72) Inventor: VASTA, Paul, J., Mckinney, TX 75070 (US); PERCOCO, Louis, Plano, TX 75025 (US); BRONSON, Dwight, G., Coppell, TX 75019 (US); WIGGINTON, Robert, E., McKinney, TX 75070 (US)
(74) Representative: Lawrence, John
(86) International application number: PCT/US2008/056791
(87) International publication number: WO 2008/112853

(56) References cited:
- US-A- 3 745 995
- US-A- 4 624 249
- US-A- 5 269 745
- US-A- 6 132 362
- US-A1- 2002 004 659
- US-A1- 2003 191 466
- US-A1- 2005 059 968
- US-B1- 6 678 562
- US-B1- 6 678 562

## Description

### BACKGROUND

### FIELD OF THE INVENTION

The disclosed embodiments relate generally to medical devices and more specifically to medical devices for the treatment of bone and soft tissue injuries. The closest prior art is document US 66778562 B1, which defines the preamble of claim 1.

### BACKGROUND OF THE INVENTION

Bone fractures and soft tissue injuries often are slow to heal, requiring a long-term treatment plan designed to aid the body's natural healing process. A conventional treatment plan might call for immobilization of the injured body part, allowing healing to take place without the wear and tear of normal use. Immobilization can also be quite important since the injured body part may need to be held in the proper position to ensure that healing correctly takes place. By way of example, when dealing with a bone fracture, the bones must be held in proper alignment to enable them to grow back together, mending properly. Immobilization may also assist in the healing of soft tissue injury by allowing the injured body part to rest and by supporting the injured body part in a way that will not cause undue tightening and an associated loss of range of motion.

A variety of immobilization techniques exist, including the use of a cast, a splint, an internal fixation device (surgically implanted within the patient to fix two bones), or external fixation devices. External fixation devices may offer advantages over other conventional methods of treatment, and so are often used in the treatment of bone fractures, soft tissue injuries, and reconstructive surgery. External stabilization devices or splints are frequently used to position a fracture or osteotomy in proper alignment during the healing process. External fixation devices may also be used in a variety of clinical procedures to lengthen or shorten bone, or to delay joint replacement. Multiple bone screws are often used to provide interfragmental compression or to attach plates which provide compression, prevent displacement of bone or tissue fragments and support the fractured bone or tissue fragments during healing. These screws, wires and/or pins are placed through one or both cortices of bone to properly position and align the fracture, non-union or osteotomy. Delayed union or non-union bone fractures are typically considered injuries that have not made satisfactory progress towards healing. External fixation devices allow easy access to wounds, adjustment during the course of healing and often allow more functional use of the fractured limb. Such devices may be used in place of a conventional cast.

In some patients, such as diabetics, bone fractures are especially slow to heal. In other instances, there is a desire to simply accelerate the natural healing process for the sake of convenience. Regardless, techniques have been sought that improve healing of bone fractures and accelerate improvement of soft tissue injuries. One such technique involves the use of Pulsed ElectroMagnetic Field therapy. Pulsed ElectroMagnetic Field (PEMF) therapy has been used to treat therapeutically resistant problems of the musculoskeletal system. Examples of instances where PEMF therapy may be useful include treatment of non-union bone fractures and delayed union bone fractures. PEMF therapy has also been used for treatment of various types of soft body tissue injuries.

PEMF therapy has been satisfactorily used as part of a treatment regimen for spinal fusion, failed arthrodeses, osteonecrosis, and chronic refractory tendonitis, decubitus ulcers and ligament, tendon injuries, osteoporosis, and Charcot foot. During PEMF therapy, an electromagnetic transducer coil is generally placed in the vicinity of the musculoskeletal injury or soft body tissue injury (sometimes referred to as the "target area" or "injured region") such that pulsing the transducer coil will produce an applied or driving field that penetrates to the underlying damaged bone or other body tissue. PEMF therapy typically uses low-energy, time varying electromagnetic fields.

PEMF therapy works by simulating one of the body's own natural processes for healing. When human bone is bent or broken, it generates an electrical field. This low level electrical field activates the body's internal repair mechanism, stimulating bone growth. In some patients, this healing process is impaired or absent, however, and the fracture may not mend properly. The bone growth stimulation provided by PEMF technology may assist in treating such fractures. PEMF therapy uses a low level electromagnetic field applied at the fracture site in order to reproduce or enhance the body's natural healing process. The electromagnetic field stimulates bone growth at the site of injury in the same manner that the body's natural healing process would, improving healing of the bone. PEMF also appears to similarly speed the recovery of soft tissue injuries.

US Patent No. 5 269 745 discloses an apparatus for regulating tissue growth comprising a magnetic field generator. The field generator comprises coils which are strapped to target tissue in use.

### SUMMARY OF THE INVENTION

While both external fixation devices and PEMF devices are independently useful conventional tools for healing bone and soft tissue injuries, used together they can work in synergy to improve the overall healing effect. The present application discloses embodiments that combine the useful aspects of external fixation devices and PEMF devices. Specifically, disclosed embodiments utilize an external fixation support frame that encompasses the injured region, in conjunction with an integrated PEMF device. The disclosed embodiments typically integrate an electromagnetic coil within the frame structure of the external fixation device, allowing generation of a PEMF field around the injured region.

According to the invention, a device for treating an injured body part is set out in claim 1.

The external fixation device of the disclosed embodiments typically uses radial elements to encompass the injured region. The term "encompass" is meant to include embodiments that entirely or partially surround the injured region. Besides encompassing the injury, providing support and stability to the injured body part, the frame of the external fixation device provides a closed loop path for the PEMF coil, so that the PEMF effect can be generated. The encompassing external frame is generally removably attached to the patient's bone about the injured region. Typically, a connector attaches the frame to the bone, and could be used to removably affix the frame of the external fixation device to the bone at one or more locations. The connector may, for example, comprise bone screws, pins, wires, or any other manufactured elements.

Beneficially, the PEMF field of the disclosed embodiments may help to reduce the risk of infection at the point of attachment, providing synergistic benefits. Attaching such an external fixation device to the bone proximate to an injured region allows the external fixation device to stabilize, compress, and/or distend the injured region, as needed to assist in healing. And by encompassing the injury, the external fixation device may shield the injury from any potential sources of aggravation.

By its very nature, the encompassing external fixation frame provides beneficial support while minimizing the introduction of any unwanted stresses to the injured region. By encompassing and encircling (either fully or partially) the injured region, the external fixation frame generally maintains a common point of origin of rotation with the bone, thus holding the bone in a natural position. Since the bone is supported on opposing sides, no unwanted stresses would be introduced. This is particularly useful, since most conventional external fixation devices are linear in nature, attaching only to one side of the bone. Such one-sided support means that whenever the bone is rotated into position, compression stresses are introduced at the bone gap. These compression stresses would arise due to the fact that the fixation device is rigidly joined to the bone, but has a pivot point of rotation different from that of the bone. Thus, when a conventional fixation device rotates a bone, it introduces unwanted compressive stresses upon the bone. Naturally, avoiding any such unwanted compression stresses may accelerate the healing process. Therefore, the use of an encompassing external fixation device aids in the healing of an injured region.

Furthermore, by encompassing the injured region, the external fixation frame of the disclosed embodiments provides a secure platform for mounting the PEMF coils in proximity to the injured region. This configuration provides for substantial coverage of the injured region with the PEMF electromagnetic fields that will stimulate healing. By encircling the injured region with coils, a PEMF field of more uniform density can be targeted to interact holistically with the injured region. The PEMF field of the disclosed embodiments encompasses the injured region, interacting radially with the injury to provide targeted healing from all directions. This targeted approach may accelerate the healing effect of PEMF treatment. PEMF therapy would typically be located near any fracture and/or area of tissue damage. The PEMF field may increase bone mineral density throughout the fracture site, while stimulating tissue growth in the entire region surrounding the fracture. By working together, the encompassing external fixation frame and integrated PEMF coils may decrease the time necessary for healing.

Depending upon the specific injury being treated, the geometry of the external fixation frame and the PEMF coil may be configured to maximize their effectiveness in treating the injury. By way of example, a small fracture of a long bone, such as the tibia, might be treated using an embodiment with a single ring serving as the external fixation frame, encircling the tibia in proximity to the injured region. In such an embodiment, a coil would typically be integrated within the frame, forming a loop that would project a PEMF field all around the injured region

On the other hand, a more substantial fracture in a long bone like the tibia, femur, or humerus might be treated using an external fixation frame with two parallel rings separated sufficiently to encompass the entire injured region between them. The two rings would be linked together by at least two bridging elements, which would hold the rings apart in parallel planes at a fixed distance. Again, the PEMF coil would typically run throughout the frame, with a ring of coils corresponding to each of the external fixation frame rings, and coil lengths corresponding to the bridging elements that would electrically link the two rings of coils (providing a closed loop).

Alternative embodiments could also be employed, with geometries corresponding to the specific body part/injury being treated. By way of a specific example, the treatment of Charcot foot could be improved by utilizing an external fixation frame designed to encompass the affected foot in such a way as to stabilize the foot while focusing PEMF treatment on the specific regions of the foot in need of healing. For Charcot foot, the rear and mid-foot regions need specific assistance promoting bone fusion. Thus, the external fixation frame may be formed with a U-shaped (alternatively described as horseshoe shaped, arch-shaped, or parabolic) bottom element, located in substantially the same plane as the bottom of the foot being treated, and a semi-circular (arch-shaped or parabolic) top element, oriented in a plane substantially parallel to that of the bottom element located a fixed distance above the bottom element (in proximity to the ankle region). These two elements would be held apart in substantially parallel planes by two or more linking/bridging elements, rigidly joining the top element to the bottom element.

In one such example, the arch shape of the top element would generally align with the arch of the bottom element's U-shape, and the linking elements would angle up from near the front of the bottom element, angling up and back, and would rigidly attach to the front of the top element. Alternatively, the top and bottom elements of the frame could be oriented in opposite directions, such that the open portions of each element would face each other (although in different, parallel planes), and the linking elements could project upward and forward from the back of the bottom element. Regardless, a continuous loop of PEMF coil would be integrated within the frame to encompass the injured region of the foot, tracing a path along the entire arch of the top element, down one of the linking elements, around the arch of the U-shaped bottom element, back up the opposite linking element to form a closed loop circuit. A control element may be attached (or possibly integrated) to the fixation frame, such that the control unit can power the PEMF coil appropriately to generate a PEMF electromagnetic wave field. Such an example may provide effective treatment for Charcot foot, or some other such foot injury, by providing encompassing support (immobilizing the injured region), by shielding the injured foot from further injury, and by providing a PEMF field that can accelerate healing of bone fractures and soft tissue injuries.

### BRIEF DESCRIPTION OF THE DRAWINGS

Embodiments are illustrated by way of example in the accompanying figures, in which like reference numbers indicate similar parts, and in which:
FIGURE 1 provides a perspective view of a single ring external fixation frame with embedded PEMF coil, located to encompass an exemplary bone around the site of fracture;
FIGURE 2 provides a perspective view of an embodiment with two parallel rings, encompassing an exemplary injured region of a bone;
FIGURE 3 provides a perspective view of an external fixation frame configured to encompass a foot, such as for treatment of Charcot foot; and
FIGURE 4 illustrates an exemplary PEMF waveform.

### DETAILED DESCRIPTION OF THE EMBODIMENTS

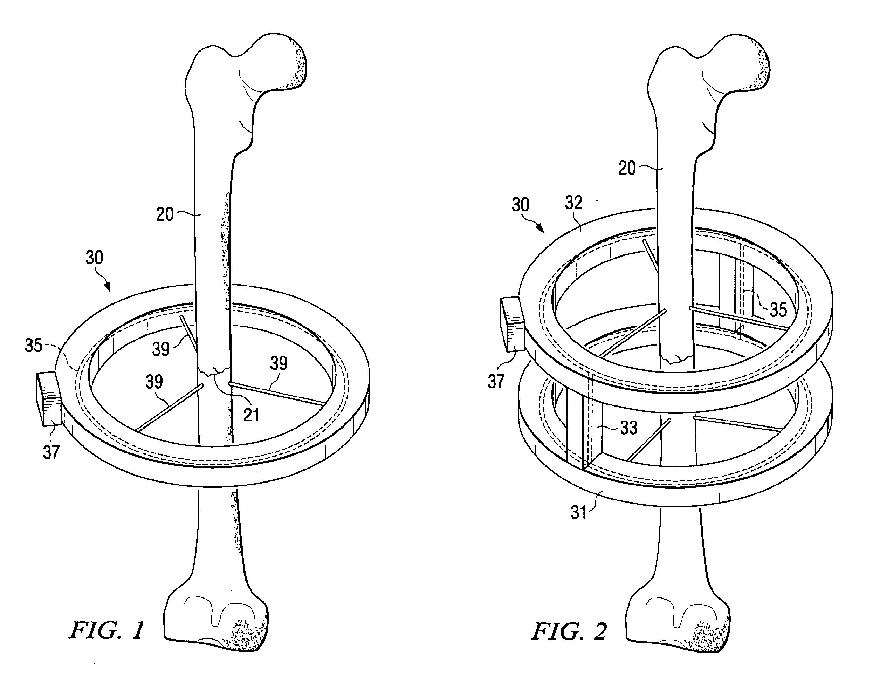

FIGURE 1 demonstrates a disclosed embodiment having a single ring that serves as the fixation frame 30. The ring of the fixation frame 30 encircles the injured region 21 of a bone 20, encompassing the entire target area. While the fixation frame 30 may be made of any suitable material that is sufficiently strong and lightweight, in the embodiment of FIGURE 1 the fixation frame 30 may be comprised of molded carbon-fiber impregnated resin. Of course, the fixation frame may be comprised of other moldable materials or even non-moldable materials. In addition to providing durable, lightweight stability and support, such a frame 30 would also have radiolucent properties, making further treatment and diagnosis simpler. In addition, using a radiolucent material for the frame 30 ensures that the frame 30 will not block or interfere with PEMF waves. It should be noted that while the ring fixation frame 30 shown in FIGURE 1 is circular in shape, there are several possible geometries that would function to encompass the injured region 21. By way of example, the ring fixation frame 30 could employ an elliptical shape, a rectangular or square shape, a triangular shape, etc, and all such encompassing geometric shapes are intended to be included within the scope of the term "ring." The embodiment of FIGURE 1 employs a circular shape because the lack of sharp corners tends to make it more ergonomical and durable in actual use.

The fixation frame 30 is releasably attached to the bone 20. Typically, connectors could be surgically attached to the bone, providing anchor points at which to attach an external fixation frame. Again, said connectors may, for example, comprise bone screws, pins, wires, or any other manufactured elements. In the embodiment of FIGURE 1, the fixation frame 30 is typically attached using a plurality of pins 39. It is to be appreciated that any number of pins, or other connectors, may be used in conjunction with tension wires to removably attach the fixation frame. The fixation frame 30 may be releasably attached to bone using connectors (either integrated within the frame 30 or separate and attaching to the frame 30). In this particular embodiment, the pins thread into the bone and the wires are drilled through the bone to maintain tension with bone.

A PEMF coil 35 would typically be integrated with the frame 30. While the coil 35 could be integrated by being externally mounted to the fixation frame 30, in the embodiment of FIGURE 1, the coil 35 is embedded within the frame 30 itself (such that the frame 30 is formed around the coil 35 in the molding process). By embedding the coil 35 within the frame 30, the coil 35 is protected from possible damage, resulting in a more durable device. Such a unified structure also provides a smoother profile, which may be more convenient for the patient. When embedding the coil 35 within the frame 30, the radiolucent nature of the frame 30 material becomes more important, since it should not act to block or shield the PEMF electromagnetic waves generated by the coil 35.

The coil 35 may produce a PEMF signal field when energized, similar to that of the Physio-Stim.RTM. or Spinal-Stim.RTM. devices offered by Orthofix. Background information regarding PEMF field generation and exemplary PEMF devices may be found by referring to co-owned Patent nos. 5,743,844; 6,132,362; 6,261,221; and 6,678,562. Typically, a PEMF signal field could be generated when a control unit 37 provides pulsing current to the coil 35 at predetermined intervals. Healthcare professionals may determine a regimen of PEMF stimulation depending upon the injury at issue, and this regimen can then be translated into a PEMF program for the control unit 37, either during manufacture or subsequently. In accordance with the PEMF program, the control unit 37 would supply current to the coil 35, thereby controlling the PEMF signal field in terms of energization time, deenergization time, and duty cycle or repetition rate.

The size and configuration for these transducer coils 35 may vary widely depending on the application. For example, larger coils 35 may be used to treat larger areas. Each transducer coil 35 may also be wound in a variety of configurations, depending on the nature of the clinical application and/or treatment site. For example, it may be advantageous to treat an area including a femur bone by using a single transducer coil 35 having approximately a 102 mm (four inch) radius and height. On the other hand, a smaller area, such as that including a tibia bone, may be suitably treated using a 76 mm (three inch) coil 35. As another example, one or more transducer coils 35 could be positioned to provide PEMF at or near fracture or osteotomy 21, and/or to an area of tissue damage. In some applications, it may also be advantageous for coils 35 to be disposed near connectors 39 to, for example, prevent the connectors 39 from loosening and/or to prevent infection.

In addition, the transducer coil 35 may include a single set of primary windings, or two or more primary windings in parallel layered on top of each other. Transducer coils 35 may be formed from commercially available eighteen gauge wire, for example. In one embodiment, transducer coils 35 may be wound according to the winding schedule: 1 layer.times.5 turns.times.20 American Wire Gauge (AWG) and each have a resistance of 0.32 ohms and an inductance of 25.4 mH. For some applications, transducer coils 35 may be wound according to a different winding schedule, for example, 2 layers.times.7 turns.times.20 AWG. And while the coil 35 is generally referred to in the singular for descriptive purposes, a plurality of coils in parallel or in series may serve the role of the PEMF coil 35 by generating the PEMF signal field, depending upon specific device configuration and needs.

Regardless of the coil 35 configuration used, the coil 35 should be structured so that it will produce a PEMF field encompassing the target area when it is energized by the control unit 37. The control unit 37 typically attaches to the frame 30 and connects to the coil 35 electrically. The control unit 37 includes access to a power source (such as a battery, or potentially an AC outlet) for activating the coil 35 in order to produce a PEMF field, as well as circuitry that controls the operation of the coil 35 (specifically, the pulse pattern for wave emissions). By way of example, the electrical circuitry of the control unit 37 may provide bi-phasic current to the coil 35 at predetermined intervals, thereby activating the PEMF output signal.

The control unit 37 would typically energize and de-energize the PEMF coil 35 in an appropriate pulsing pattern to generate a PEMF field. By way of example, the control unit 37 might output two programmed pulse trains. Each of the two pulse trains would typically provide ninety-nine pulses that in one embodiment collectively last 25,740 microseconds. Each of the pulse trains would be offset, such that when one pulse is high the other pulse would be low. Such alternating flow would switch the current on and off at the proper times to provide the desired recovery phase of operation. In one example, therefore, the first signal may be ON for 67 microseconds and OFF for 195 microseconds. Accordingly, the second pulse train would include a 195 microsecond ON period followed by a 67 microsecond OFF period. The two pulse trains may be provided every 667,000 microseconds.

Typically, at the beginning of the two pulse trains, the first pulse would be a shorter pulse than the other pulses in the same pulse train. Thus, if the first pulse train includes 65 microsecond on and 195 microsecond off times, then the first pulse of the first pulse train may be preferably 32.5 microseconds. This first short pulse sets up the magnetic field for the PEMF stimulation therapy signal in a single-winding coil 35. By turning on the drive circuitry for one-half pulse, energization of the magnetic field takes place to set the PEMF magnetic field away from zero. With this setting, the next pulse on the lower train would turn on for 195 microseconds. This sets the current so that the drive flyback energy would go in a negative direction. This would cause the remaining cycling to flow from an initial negative direction. The current would then ramp up through zero and increase from a negative number through zero to a positive number during the pulse. Thus, because the current is changing in the inductor that the PEMF transducer coil forms, a constant electrical field would be induced. The first half pulse would establish the constant PEMF field for delivering the desired PEMF therapy signal.

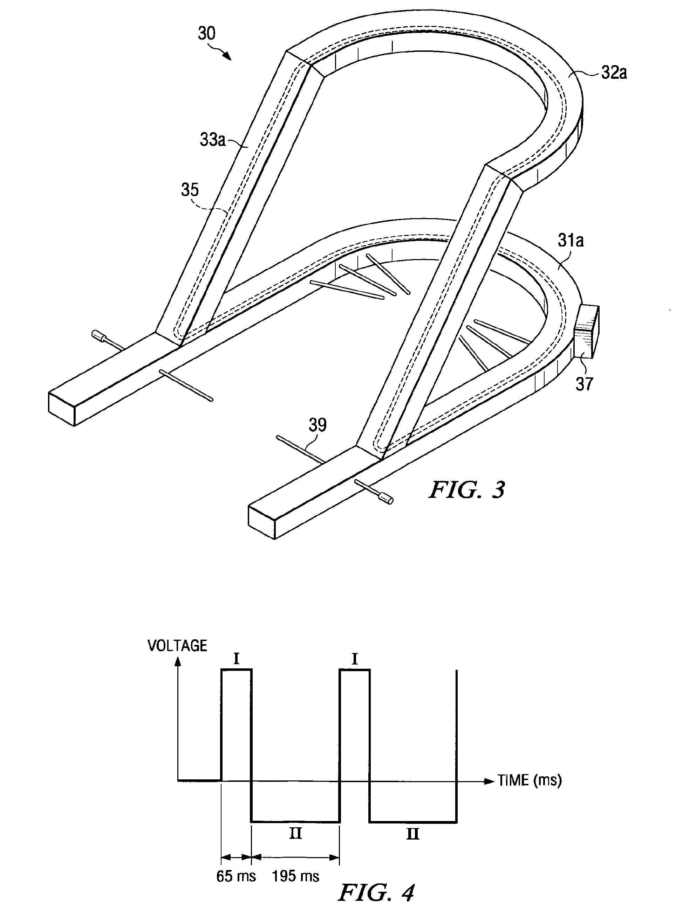

An exemplary PEMF waveform that might be generated when the control unit 37 energizes the coil 35 of the present embodiment may be seen in **FIGURE 4**. Note that the high voltage is one-third the duration and three times the magnitude of the low voltage. FIGURE 4 shows the PEMF stimulation signal output from PEMF coil 35, which includes a transformer pulse positive portion I followed by transformer pulse negative portion II. Pulse positive portion I has a duration of approximately 65 microseconds. Pulse negative portion II has a duration of approximately 195 microseconds. The positive voltage level for transformer pulse positive portion I is approximately three times the negative voltage level for pulse negative portion II. The areas of the portions I and II, therefore, are approximately equivalent. And in some applications, the PEMF signal may include a burst of pulses that may be followed by an inter-burst period. Using PEMF waveforms such as these may provide, for example, an increase in bone density, because bone tissue appears to be responsive to various harmonic content within an electrical signal.

The coil configuration, control unit specifications, and pulse waveforms discussed herein are merely illustrative, providing a specific example. A person skilled in the art field will appreciate and understand alternative configurations, specifications, and waveforms that would generate an appropriate PEMF field. Specific PEMF fields needed for particular injuries may accordingly be determined and set by medical professionals. Regardless of the specific features, the control unit 37 will supply current to the PEMF coil 35 using a pulse pattern, in such a way as to generate a PEMF signal field that will encompass and penetrate the injured region.

Thus, in practice the ring fixation frame 30 would be removably attached to the bone 20 using connectors 39, such as pins, bone screws, etc., so that it would encircle the injured region 21. Typically a plurality of such connectors would removably attach the ring fixation frame 30 in place relative to the bone at various locations, depending upon the geometry of the ring frame 30, the location of the injured region and the specific body part at issue, and the amount of stability needed. The control unit 37 would energize the coils 35 embedded within the frame 30, producing a PEMF field that would encompass and radially penetrate the injured region 21 to accelerate healing. Treatment would typically be administered daily for a duration determined by medical professionals, usually within a range of two to six hours of PEMF wave exposure.

The embodiment of **FIGURE 2** is similar in operation to the single ring embodiment of FIGURE 1, but it employs a fixation frame 30 with two rings spaced apart in parallel planes in order to provide an encompassing frame/PEMF field over a larger area. The frame 30 of FIGURE 2 has a lower ring 31 and an upper ring 32. The upper ring 32 is rigidly attached to the lower ring 31 by two or more longitudinal bridging elements 33, such that the upper ring 32 is generally parallel to the lower ring 31 of the frame 30 and the upper ring 32 is spaced a set distance away from the lower ring 31 (determined by the length of the bridging elements 33). Such a frame 30 configuration would typically be used for larger fractures in long bones such as, for example, the tibia, femur, or humerus. The frame 30 would typically be releasably attached to the bone in such a way as to encompass the injured region.

Depending upon the body part and location of fracture, alternative frame 30 configurations may be useful to ensure that the injury is fully encompassed. By way of example, one or more of the rings could instead be only partial segments, forming an arch or parabolic shape. For example, the upper element 32 could be an arch with its open end facing forward (and its rounded end facing the rear), and the lower element 31 could be an arch with its open end facing the rear (and its rounded end facing forward). The bridging elements 33 could then rigidly link the open ends of the upper element 32 with the open ends of the lower element 31. Additionally, the rings of the upper element 32 and the lower element 31 may not be parallel. And certainly, more than two rings could be linked together, depending upon the length of the injured region needing treatment.

The fixation frame 30 of FIGURE 2 encompasses the bone 20, such that the injured region is located within and encircled by the frame 30. As noted above, the frame 30 need not fully encircle the injured region to encompass the bone. Rather, arc and parabolic shapes would also encompass the bone. The coil 35 is once again embedded within the frame 30 as a closed loop running around the lower ring element 31, up one of the bridging elements 33, around the upper ring element 32, and down the other bridging element 33. In this way, when energized, the coil 35 may produce a PEMF field that encompasses the injured region, with electromagnetic waves radially penetrating and saturating the injury to facilitate healing. Again, the control unit 37 energizes the coil 35 to produce the PEMF signal field. Details regarding the coil, control unit, and their interaction producing a PEMF signal field would generally track the earlier description set forth above.

**FIGURE 3** illustrates an example specifically designed to assist with Charcot foot. Such examples may be particularly useful for the treatment of diabetics, who have a tendency to develop Charcot foot and who have difficulty healing bone fractures. The frame 30 of the example of FIGURE 3 encompasses the foot, both stabilizing the entire foot and locating the PEMF coil 35 about the foot to provide for fall field coverage and saturation of the injured region. The frame 30 is typically constructed of two arch-shaped elements. The bottom element 31a is substantially arch shaped, and encircles the foot substantially in the same plane as the bottom of the foot such that the toes of the patient's foot would face toward the open end of the arch-shaped bottom element 31a, while the heel would be entirely encircled by the rounded portion of the arch-shaped bottom element 31a. A parabolic arch shape is typically employed for the bottom element 31 a since it may encompass the entire foot with a fairly narrow profile while leaving the toes accessible.

The top element 32a is also arch-shaped, although it is generally smaller and/or more truncated than the bottom element 31a (such that it approximates a semi-circle and does not extend as far away from its rounded end). In the example shown in FIGURE 3, the rounded end of the top element 32a is generally aligned with the rounded end of the bottom element 31a, with both elements' open ends (of their arch shape) facing forward. The top element 32a is generally located above the bottom element 31a and in a plane substantially parallel to that of the bottom element 31a. Two linking elements 33a rigidly attach the top element 32a in place with respect to the bottom element 31a. While the linking elements 33a could simply be vertical supports, in the example of FIGURE 3 the linking elements 33a are angled. The linking elements attach to the front edges of the arch-shaped top element 32a and angle downward and forward to attach to the bottom element 31 a at a location disposed towards the front edges of the bottom element 31a. This geometry is designed to encompass the foot in such a way as to focus the PEMF signal on the rear and mid-foot regions, where joint fusion is most desired for Charcot Foot. The frame 30 is typically releasably attached to the injured foot.

As persons skilled in the art field will appreciate, alternative embodiments are also possible. For instance, with the top element 32a and the bottom element 31a oriented as described above, the linking elements 33a could angle in the opposite direction, rigidly attaching to the front edges of the top element 32a and projecting downward and backwards to attach near the rear, rounded portion of the bottom element 31a. In another alternative example, the top element 32a could be oriented with its rounded end facing forward and its open end facing back towards the heel of the foot, with the linking elements 33a attaching to the open ends of the top element 32a and extending downward and backward to rigidly attach to the bottom element 31a. Alternatively, the top element 32a could be a ring that completely encircles the ankle region of the patient's foot. The specific configuration of the frame 30 may be selected depending upon the region of the patient's foot that needs to receive the focus of the PEMF treatment (since the frame configuration will determine the placement of the PEMF coil based on the connection of the elements that will form a closed loop).

Regardless of the configuration selected for the frame 30, the coil 35 would typically be integrated with the frame 30, forming a closed loop (allowing current flow through the coil 35). While the coil 35 may be mounted upon the frame 30 externally, in the example of FIGURE 3 the coil 35 is embedded within the frame 30. A control element 37 may either be integrated with the frame or externally attached. The control element 37 interacts electrically with the coil 35, powering the coil 35 to produce a PEMF field (as similarly described above in more detail for other embodiment variants). So when the disclosed example of FIGURE 3 is used on a patient's foot, the control element 37 powers the coil 35 to produce a PEMF field that encompasses the foot, focusing the PEMF signal on the appropriate injured region to speed healing, while the frame 30 stabilizes the foot.

In all of the specific disclosed embodiments, the frame 30 employs radial elements in order to encompass the injured region. By way of example, the frame 30 may be entirely constructed of one or more rings (whether circular, elliptical, square, triangular, or some other such encompassing shape) that encircle the injured region, or it may be constructed of portions of rings (such as arch shapes, parabolas, or V-Shapes) linked together. Regardless, the frame 30 employs a radial design in order to effectively encompass the injured region. This provides the injured region with good support and stability, while also allowing the PEMF field generated by the integrated coil 35 to fully encompass the injured region. Since the coil 35 radially encircles the injured region, the electromagnetic PEMF waves radially penetrate the injury, ensuring that the site of the injury is fully saturated. This maximizes the healing effect of the PEMF field. The PEMF field in the various embodiments would generally be formed employing substantially similar techniques, varying primarily in the location of the PEMF field based upon the geometrical configuration of the coil 35. There may also be a synergistic healing effect between the frame 30 and the PEMF coil 35, since the frame 30 acts to immobilize the injured region without introducing unwanted stresses, such that the injured tissue is placed in a restful position conducive to the healing effects of the PEMF. Likewise, the PEMF may act to inhibit infection at the site of attachment between the frame 30 and the bone. Working in unison, the various elements of the disclosed embodiments assist in mending bone fractures and healing soft tissue injuries.

While various embodiments in accordance with the principles disclosed herein have been described above, it should be understood that they have been presented by way of example only, and not limitation. Thus, the breadth and scope of the invention(s) should not be limited by any of the above-described exemplary embodiments, but should be defined only in accordance with the claims. Furthermore, the above advantages and features are provided in described embodiments, but shall not limit the application of such issued claims to processes and structures accomplishing any or all of the above advantages.

## Claims

1. A device for treating an injured body part (20) comprising:
a frame (30);
a coil (35) integrated with at least a portion of the frame (30), the coil (35) forming a closed electrical loop;
a control element (37); and
at least one connector (39) for removably attaching the frame (30) to the injured body part (20);
wherein the control element (37) interacts electrically with the coil (35) to induce the coil to generate a PEMF signal; wherein
the coil is operable to at least partially surround the injured body part; **characterised in that**
the frame (30) is an encompassing frame comprising a radiolucent material, wherein the radiolucent material minimises the frame's interference with the PEMF signal and the PEMF signal is permitted to interact radially with the injured body part from all directions.

2. A device as in claim 1, wherein one selected from:
(i) the control element (37) supplies power to the coil;
(ii) the frame (30) is comprised of a moldable material;
(iii) the frame (30) is comprised of a non-moldable material;
(iv) the frame (30) comprises a ring encircling the injured body part (20);
(v) the frame (30) comprises a ring partially encircling the injured body part (20); and
(vi) the frame (30) comprises a plurality of rings (31, 32) and a plurality of bridging elements (33), wherein the plurality of rings (31, 32) are located in generally parallel planes and are rigidly attached together by the bridging elements (33).

3. A device as in claim 1, wherein the frame (30) comprises: a bottom element (31a), substantially parabolic in shape; a top element (32a), substantially parabolic in shape; and
a plurality of linking elements (33a), rigidly attaching the top element (32a) and the bottom element (31a) in such a manner that the top element (32a) is located above and substantially parallel to the bottom element (31a).

4. A device as in claim 3, wherein the rounded end of the top element (32a) is generally aligned with the rounded end of the bottom element (31a), such that the open end of both the top element (32a) and the bottom element (31a) face substantially the same direction.

5. A device as in claim 4, wherein the bottom element (31a) is located in substantially the same plane as the bottom of a patient's foot.

6. A device as in claim 4, wherein the plurality of linking elements (33a) rigidly attach to the front open edges of the top element (32a) and angle forward and downward to rigidly attach to the bottom element (31a).

7. A device as in claim 1, wherein one selected from:
(i) the PEMF signal produces a low-energy, time-varying electromagnetic field;
(ii) the frame (30) is comprised of molded carbon-fiber impregnated resin, and wherein the coil (35) is embedded within the frame (30); and
(iii) the PEMF signal generated by the coil (35) encompasses the injured body part.

## Patentansprüche

1. Ein Gerät für die Behandlung eines verletzten Körperteils (20), das Folgendes aufweist:
einen Rahmen (30);
eine Spule (35), die in mindestens einen Teil des Rahmens (30) integriert ist, die Spule (35) bildet dabei eine geschlossene elektrische Schleife; ein Steuerelement (37); und
mindestens ein Verbindungsstück (39) zur lösbaren Befestigung des Rahmens (30) am verletzten Körperteil (20);
wobei das Steuerelement (37) elektrisch mit der Spule (35) zusammenwirkt, um die Spule zur Erzeugung eines PEMF-Signals zu veranlassen;
wobei die Spule in der Lage ist, zumindest teilweise das verletzte Körperteil zu umgeben; **dadurch gekennzeichnet, dass**
der Rahmen (30) ein umgreifender Rahmen ist, der ein strahlendurchlässiges Material aufweist, wobei das strahlendurchlässige Material die Störung des PEMF-Signals durch den Rahmen minimiert und das PEMF-Signal radial aus allen Richtungen auf das verletzte Körperteil einwirken kann.

2. Ein Gerät gemäß Anspruch 1, wobei einer der folgenden Punkte gilt:
(i) das Steuerelement (37) liefert Strom an die Spule;
(ii) der Rahmen (30) weist ein formbares Material auf;
(iii) der Rahmen (30) weist ein nichtformbares Material auf;
(iv) der Rahmen (30) weist einen Ring auf, der den verletzten Körperteil (20) umgibt;
(v) der Rahmen (30) weist einen Ring auf, der den verletzten Körperteil (20) teilweise umgibt; und
(vi) der Rahmen (30) weist eine Vielzahl von Ringen (31, 32) und eine Vielzahl von Verbindungselementen (33) auf, wobei die Vielzahl der Ringe (31, 32) im Allgemeinen auf parallelen Ebenen angeordnet und über die Verbindungselemente (33) fest miteinander verbunden ist.

3. Ein Gerät gemäß Anspruch 1, wobei der Rahmen (30) Folgendes aufweist: ein Unterteil (31 a), das im Wesentlichen parabelförmig ist; ein Oberteil (32a), das im Wesentlichen parabelförmig ist; und
eine Vielzahl von Verbindungselementen (33a), die das Oberteil (32a) und das Unterteil (31 a) fest miteinander verbinden, in einer Weise, dass das Oberteil (32a) über dem und im Wesentlichen parallel zum Unterteil (31 a) angebracht ist.

4. Ein Gerät gemäß Anspruch 3, wobei das abgerundete Ende des Oberteils (32a) im Allgemeinen zum abgerundeten Ende des Unterteils (31a) ausgerichtet ist, so dass das offene Ende sowohl des Oberteils (32a) als auch des Unterteils (31 a) im Wesentlichen in dieselbe Richtung zeigen.

5. Ein Gerät gemäß Anspruch 4, wobei das Unterteil (31 a) im Wesentlichen in derselben Ebene liegt wie die Fußsohle eines Patienten.

6. Ein Gerät gemäß Anspruch 4, wobei die Vielzahl der Verbindungselemente (33a) fest mit den vorderen offenen Rändern des Oberteils (32a) verbunden sind und nach vorne und unten abgewinkelt sind, um sich fest mit dem Unterteil (31a) zu verbinden.

7. Ein Gerät gemäß Anspruch 1, wobei einer der folgenden Punkte gilt:
(i) das PEMF-Signal erzeugt ein energiearmes, zeitlich veränderliches elektromagnetisches Feld;
(ii) der Rahmen (30) weist ein geformtes mit Kohlefaser imprägniertes Harz auf, wobei die Spule (35) in den Rahmen (30) eingebettet ist; und
(iii) das PEMF-Signal, das von der Spule (35) erzeugt wird, umringt den verletzten Körperteil.

## Revendications

1. Un dispositif de traitement d'une partie corporelle blessée (20) comprenant :
un châssis (30),
une bobine (35) intégrée à au moins une partie du châssis (30), la bobine (35) formant une boucle électrique fermée,
un élément de commande (37), et
au moins un connecteur (39) destiné à fixer de manière amovible le châssis (30) à la partie corporelle blessée (20),
où l'élément de commande (37) interagit électriquement avec la bobine (35) de façon à induire la bobine à générer un signal PEMF,
où la bobine est conçue de façon à au moins partiellement envelopper la partie corporelle blessée, **caractérisé en ce que**
le châssis (30) est un châssis englobant comprenant un matériau radiotransparent, où le matériau radiotransparent minimise l'interférence du châssis avec le signal PEMF et le signal PEMF est autorisé à interagir radialement avec la partie corporelle blessée à partir de toutes les directions.

2. Un dispositif selon la Revendication selon la Revendication 1, où un élément sélectionné parmi :
(i) l'élément de commande (37) fournit de l'énergie à la bobine,
(ii) le châssis (30) se compose d'un matériau moulable,
(iii) le châssis (30) se compose d'un matériau non moulable,
(iv) le châssis (30) comprend un anneau encerclant la partie corporelle blessée (20),
(v) le châssis (30) comprend un anneau encerclant partiellement la partie corporelle blessée (20), et
(vi) le châssis (30) comprend une pluralité d'anneaux (31, 32) et une pluralité d'éléments de pontage (33), où la pluralité d'anneaux (31, 32) sont situés dans des plans généralement parallèles et sont fixés les uns aux autres de manière rigide par les éléments de pontage (33).

3. Un dispositif selon la Revendication 1, où le châssis (30) comprend : un élément inférieur (31a) de forme sensiblement parabolique, un élément supérieur (32a) de forme sensiblement parabolique, et
une pluralité d'éléments de liaison (33a), fixant de manière rigide l'élément supérieur (32a) et l'élément inférieur (31 a) de telle manière que l'élément supérieur (32a) soit situé au-dessus de et sensiblement parallèle à l'élément inférieur (31a).

4. Un dispositif selon la Revendication 3, où l'extrémité arrondie de l'élément supérieur (32a) est généralement alignée avec l'extrémité arrondie de l'élément inférieur (31 a), de sorte que l'extrémité ouverte à la fois de l'élément supérieur (32a) et de l'élément inférieur (31 a) soit tournée dans sensiblement la même direction.

5. Un dispositif selon la Revendication 4, où l'élément inférieur (31 a) est situé dans sensiblement le même plan que la partie inférieure d'un pied de patient.

6. Un dispositif selon la Revendication 4, où la pluralité d'éléments de liaison (33a) sont rattachés de manière rigide aux bords ouverts avant de l'élément supérieur (32a) et s'inclinent vers l'avant et vers le bas de façon à se fixer de manière rigide à l'élément inférieur (31a).

7. Un dispositif selon la Revendication 1, où un élément sélectionné parmi :
(i) le signal PEMF produit un champ électromagnétique à faible énergie et variant dans le temps,
(ii) le châssis (30) se compose d'une résine moulée imprégnée de fibre de carbone, et où la bobine (35) est imbriquée à l'intérieur du châssis (30), et
(iii) le signal PEMF généré par la bobine (35) englobe la partie corporelle blessée.
